# EUROPEAN PATENT APPLICATION

(11) **EP 4 667 959 A1**
(43) Date of publication of application: **24.12.2025**
(21) Application number: 25182184.9
(22) Date of filing: 11.06.2025
(51) Int. Cl.: G01R 33/34, G01R 33/3415, A61B 5/00, A61B 5/08

(54) **COIL SET AND TRUNK PART COIL FOR RECEIVING A NUCLEAR MAGNETIC RESONANCE SIGNAL**

(30) Priority: 21.06.2024 JP 2024100280
(71) Applicant: FUJIFILM Corporation, Tokyo Tokyo 106-8620 (JP)
(72) Inventor: IWASAWA, Kojiro, Tokyo (JP); ARAI, Koichi, Tokyo (JP); DOHATA, Masayoshi, Tokyo (JP); OTAKE, Yosuke, Tokyo (JP); OCHI, Hisaaki, Tokyo (JP)
(74) Representative: Dehns Germany Partnerschaft mbB

(57) **Abstract**

Provided are a coil set (10, 12, 14) and a trunk part coil (22) that can easily fix a receiving coil (26) to a subject by closely attaching the receiving coil (26) to the subject. A coil set (10, 12, 14) according to the present disclosure includes: a trunk part coil (22) that has flexibility and in which a plurality of receiving coils (26) that receive a nuclear magnetic resonance signal of a subject are two-dimensionally arranged; two belts (30, 32, 130, 132) that are configured separately from the trunk part coil (22) and that fix the trunk part coil (22) to the subject; and a length adjustment mechanism (42, 142) that fixes the two belts (30, 32, 130, 132) at positions of arbitrary lengths, in which the trunk part coil (22) includes a plurality of through-holes (34A, 36A, 38A, 40A), and the two belts (30, 32, 130, 132) are each passed through any of the plurality of through-holes (34A, 46A, 38A, 40A) , and the length adjustment mechanism (42, 142) adjusts lengths of the two belts (30, 32, 130, 132) to arbitrary lengths.

## Description

### BACKGROUND OF THE INVENTION

### 1. Field of the Invention

The present invention relates to a coil set and a trunk part coil, and particularly to a technique of fixing a trunk part coil in which a receiving coil that receives a nuclear magnetic resonance signal is disposed to a subject.

### 2. Description of the Related Art

In a case of performing abdominal imaging in a magnetic resonance imaging (MRI) examination, it is necessary to fix an abdominal coil including a radio frequency (RF) receiving coil to prevent the abdominal coil from being displaced with respect to the abdomen of a patient. JP2012-152465A, JP1992-002346A (JP-H4-002346A), and JP1990-006007U (JP-H2-006007U) disclose a technique of fixing a patient to an examination table by intersecting belts and fixing the belts.

### SUMMARY OF THE INVENTION

In an MRI examination, it is desirable to closely attach an RF receiving coil to a subject in order to perform high-quality imaging in a region where the sensitivity of the RF receiving coil is high. However, since a fixing belt in the related art is fixed from an end part of an examination table, there is a problem that the RF receiving coil cannot be closely attached to a side surface of a trunk part and a gap is generated.

In addition, in the fixing belt in the related art, the fixing belt on a side opposite to a side on which a technician of the examination table is standing is far from the technician and is difficult to reach. Therefore, there is a problem in that, particularly for a subject with a large physique, a technician with a small physique may not be able to reach the fixing belt on the opposite side and needs to go around to the opposite side of the examination table.

The present invention has been made in view of such circumstances, and an object of the present invention is to provide a coil set and a trunk part coil that can easily fix a receiving coil to a subject by closely attaching the receiving coil to the subject.

In order to achieve the above object, a coil set according to a first aspect of the present disclosure comprises a trunk part coil that has flexibility and in which a plurality of receiving coils that receive a nuclear magnetic resonance signal of a subject are two-dimensionally arranged; two belts that are configured separately from the trunk part coil and that fix the trunk part coil to the subject; and a length adjustment mechanism that fixes the two belts at positions of arbitrary lengths, in which the trunk part coil includes a plurality of through-holes, and the two belts are each passed through any of the plurality of through-holes, and the length adjustment mechanism adjusts lengths of the two belts to arbitrary lengths.

In a coil set according to a first embodiment of the present disclosure, it is preferable that in the coil set according to the first aspect, the two belts intersect each other to fix the trunk part coil to the subject.

**In** a coil set according to a second embodiment of the present disclosure, it is preferable that in the coil set according to the first aspect or the first embodiment, the trunk part coil includes four through-holes.

**In** a coil set according to a third embodiment of the present disclosure, it is preferable that in the coil set according to the second embodiment, the trunk part coil includes the through-holes at four corners of the trunk part coil in a plan view.

In a coil set according to a fourth embodiment of the present disclosure, it is preferable that in the coil set according to any one of the first aspect or first to third embodiments, the length adjustment mechanism is disposed on an opposite side of the subject, with the trunk part coil interposed therebetween.

In a coil set according to a fifth embodiment of the present disclosure, it is preferable that in the coil set according to the fourth embodiment, the length adjustment mechanism is fixed to a center of the trunk part coil in a plan view.

In a coil set according to a sixth embodiment of the present disclosure, it is preferable that in the coil set according to any one of the first aspect or first to fifth embodiments, the length adjustment mechanism includes a relay connector to which a cable that outputs a nuclear magnetic resonance signal received by the plurality of receiving coils is connected.

In a coil set according to a seventh embodiment of the present disclosure, it is preferable that in the coil set according to any one of the first aspect or first to sixth embodiments, each of the two belts includes a first belt member that includes one of a pair of connecting members, and a second belt member that includes the other of the pair of connecting members.

In a coil set according to an eighth embodiment of the present disclosure, it is preferable that in the coil set according to the seventh embodiment, the coil set further comprises a breathing synchronization sensor that is disposed between the trunk part coil and the subject and that detects breathing of the subject.

In a coil set according to a ninth embodiment of the present disclosure, it is preferable that in the coil set according to any one of the first aspect or first to eighth embodiments, the trunk part coil has a rectangular shape in a plan view, and is configured to fix the subject in any of an orientation in which a long side of the rectangular shape is aligned with an axis of an examination target part of the subject and an orientation in which a short side of the rectangular shape is aligned with the axis of the examination target part of the subject.

In order to achieve the above object, a trunk part coil according to a second aspect of the present disclosure is a trunk part coil that is fixed to a subject, the trunk part coil comprising: a plurality of receiving coils that receive a nuclear magnetic resonance signal of the subject; a blanket that has flexibility and in which the plurality of receiving coils are two-dimensionally arranged; a plurality of through-holes that are disposed on the blanket and through which two belts configured separately from the trunk part coil are passed; and a length adjustment mechanism that fixes the two belts passed through the plurality of through-holes at positions of arbitrary lengths.

According to the present invention, the subject and the receiving coil can be closely attached to each other and easily fixed to each other.

### BRIEF DESCRIPTION OF THE DRAWINGS

Fig. 1 is a perspective view showing an example of an MRI apparatus to which a coil unit is applied.
Fig. 2 is a sectional schematic view taken along a line 2-2 of Fig. 1.
Fig. 3 is a perspective view of a coil set according to a first embodiment.
Fig. 4 is a perspective view showing an example of a configuration of a trunk part coil.
Fig. 5 is a top view of a trunk part coil put on an abdomen of a subject.
Fig. 6 is a schematic view viewed from an arrow A of Fig. 5.
Fig. 7 is a top view of a trunk part coil put on an abdomen of a subject by another method.
Fig. 8 is a perspective view of a coil set according to a second embodiment.
Fig. 9 is a top view of a trunk part coil put on an abdomen of a subject.
Fig. 10 is a front view of a coil set according to a third embodiment.
Fig. 11 is a top view for explaining a method of using a coil set.
Fig. 12 is a top view of a trunk part coil put on an abdomen of a subject.
Fig. 13 is a schematic view viewed from an arrow B of Fig. 12.

### DESCRIPTION OF THE PREFERRED EMBODIMENTS

Hereinafter, preferred embodiments of a coil unit according to the present disclosure will be described with reference to the accompanying drawings. In the present specification, the same components are denoted by the same reference numerals, and duplicate description thereof will be omitted as appropriate.

### [Configuration of Typical Apparatus]

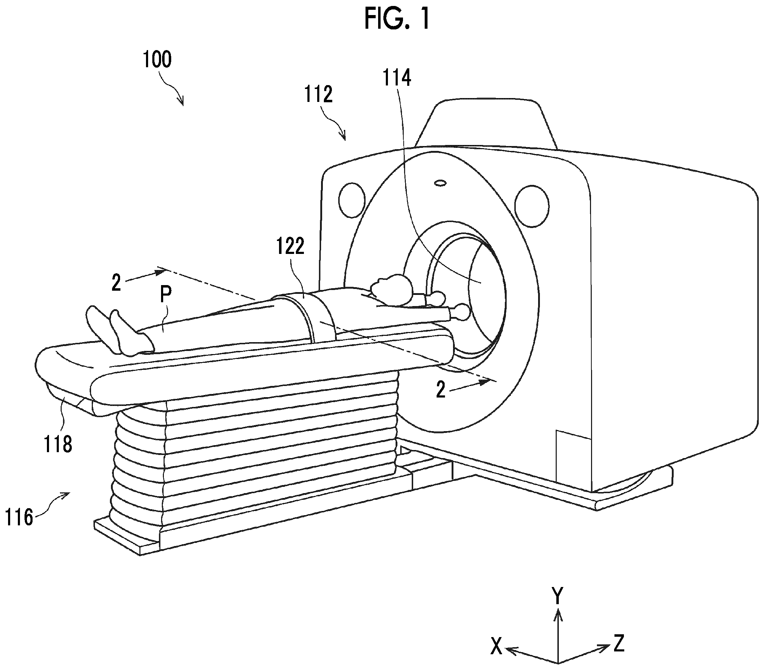

Fig. 1 is a perspective view showing an example of a typical MRI apparatus 100. As shown in Fig. 1, the MRI apparatus 100 comprises a gantry 112 and an examination table 116. The gantry 112 has a cylindrical examination (imaging) space 114. In addition, an MRI magnet (not shown) as a magnetic field generation source and various other coils (not shown) are disposed in the gantry 112.

The examination table 116 is installed to face the examination space 114 on a front side of the gantry 112. The examination table 116 comprises a top plate 118. A subject P is placed on the top plate 118 with a body axis in a horizontal direction along a longitudinal direction. The top plate 118 is configured to be movable in an X direction that is a width direction orthogonal to the longitudinal direction of the top plate 118, in a Y direction that is a vertical direction, and in a Z direction that is the longitudinal direction of the top plate 118.

The MRI apparatus 100 comprises a drive mechanism (not shown) that moves the top plate 118 in the Z direction to allow the top plate 118 to enter the examination space 114 and exit from the examination space 114. In the MRI apparatus 100, the top plate 118 is moved in the examination space 114, so that the examination target part of the subject P to be imaged placed on the top plate 118 is set to a center of a static magnetic field in the examination space 114.

A multi-channel RF receiving coil array (not shown) consisting of a plurality of RF receiving coils for receiving the nuclear magnetic resonance signal generated in the subject P is fixed by a fixing belt 122 at the examination target part of the subject P. In the example shown in Fig. 1, an abdomen of the subject P is the examination target part, and an RF receiving coil array is put on the abdomen of the subject P.

A reception cable (not shown) that outputs the nuclear magnetic resonance signal received by the RF receiving coil is connected to the RF receiving coil array. A receiving-side connector (not shown) is connected to an end part of the reception cable. The receiving-side connector is connected to an examination table-side connector of an examination table cable (not shown). As a result, the reception cable and the examination table cable are communicably connected via their respective connectors.

The examination table cable is stored in a cable storage portion (not shown) of the examination table 116. The nuclear magnetic resonance signal of the subject P received by the RF receiving coil is transmitted to a signal processing unit (not shown) via the reception cable and the examination table cable. The signal processing unit performs signal processing on the received nuclear magnetic resonance signal to convert the received nuclear magnetic resonance signal into an image signal.

### [Problems]

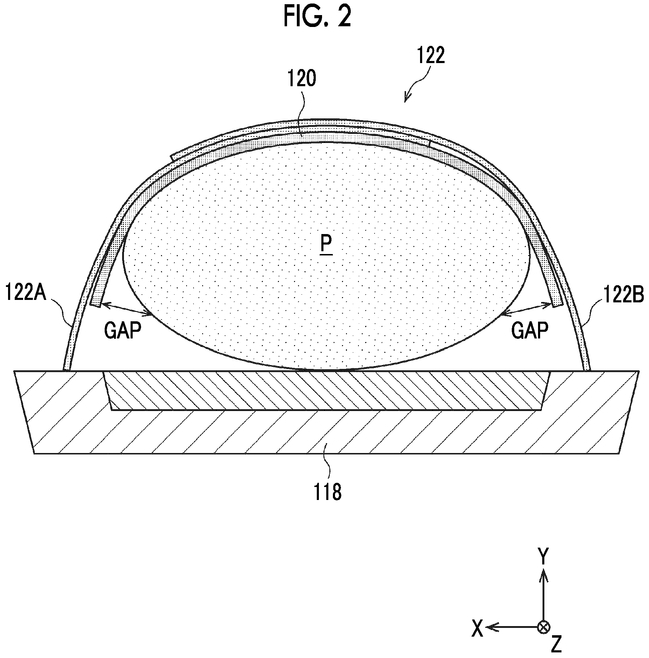

Fig. 2 is a sectional schematic view taken along a line 2-2 of Fig. 1. As shown in Fig. 2, an abdominal coil 120 including the RF receiving coil array consisting of a plurality of RF receiving coils is disposed on the abdomen of the subject P placed on the top plate 118. The abdominal coil 120 is fixed to the abdomen of the subject P by the fixing belt 122.

The fixing belt 122 includes a first fixing belt 122A and a second fixing belt 122B. One end of the first fixing belt 122A is fixed to an end part of the top plate 118 on the +X direction side, and the other end of the first fixing belt 122A is directed to the -X direction side and is placed on the abdomen of the subject P in a state of being overlapped with the abdominal coil 120. One end of the second fixing belt 122B is fixed to an end part of the top plate 118 on the -X direction side, and the other end of the second fixing belt 122B is directed to the +X direction side and is placed on the abdomen of the subject P in a state of being overlapped with the first fixing belt 122A.

As shown in Fig. 2, a gap is generated between the abdominal coil 120 and the subject P on both sides of the subject P in the X direction. Therefore, there is a problem in that the sensitivity of the RF receiving coil is reduced and high-quality imaging cannot be performed. In addition, in a case where the technician is standing on, for example, the -X direction side, the technician may not be able to reach the first fixing belt 122A on the +X direction side, and there is a problem that it takes time and effort for the technician to put the abdominal coil 120 on the subject P.

### [First Embodiment]

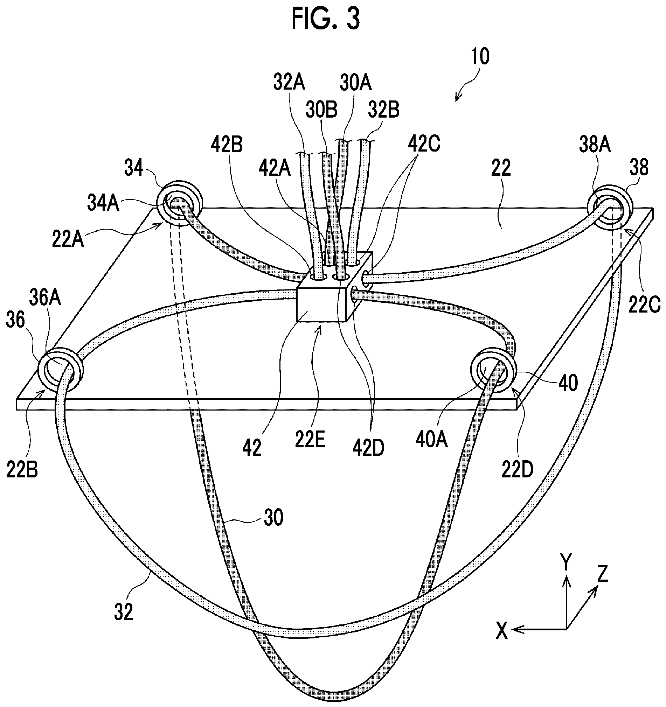

Fig. 3 is a perspective view of a coil set 10 according to a first embodiment. The coil set 10 has a mechanism for fixing the RF receiving coil array to the abdomen of the subject P.

As shown in Fig. 3, the coil set 10 comprises a trunk part coil 22 and two belts 30 and 32.

The two belts 30 and 32 are configured as separate bodies that can be separated from the trunk part coil 22. The belt 30 is a flexible string-like member comprising an end part 30A that is one free end and an end part 30B that is the other free end. The belt 32 is a flexible string-like member comprising an end part 32A that is one free end and an end part 32B that is the other free end. The belts 30 and 32 are made of a synthetic resin, leather, rubber, or metal.

The trunk part coil 22 has a rectangular shape in a plan view (in Fig. 3, a view in the Y direction). Here, a side along the X direction is a long side of the rectangular shape, and a direction along the Z direction is a short side of the rectangular shape.

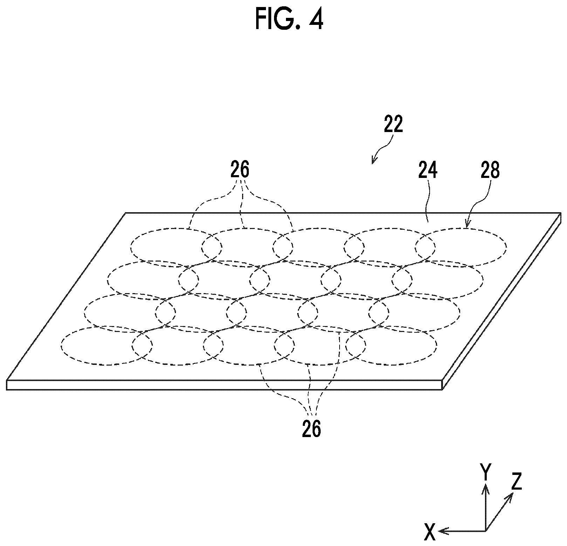

Fig. 4 is a perspective view showing an example of a configuration of the trunk part coil 22. As shown in Fig. 4, the trunk part coil 22 includes a blanket 24 and a plurality of RF receiving coils 26 (an example of a "plurality of receiving coils").

The blanket 24 is a container that covers the periphery of the plurality of RF receiving coils 26. The blanket 24 has flexibility and is formed in a bag shape by sewing or bonding an end part of a sheet-like material cut into one piece. The material of the blanket 24 may be a urethane-based resin such as polyurethane, a polyamide synthetic resin such as nylon, or the like.

The RF receiving coil 26 functions as a detector that receives a nuclear magnetic resonance signal. The RF receiving coil 26 is a circular loop coil having a diameter of about 10 cm to 15 cm in a plan view. The plurality of RF receiving coils 26 are two-dimensionally arranged inside the blanket 24. The trunk part coil 22 shown in Fig. 4 comprises an RF receiving coil array 28 consisting of a total of 20 RF receiving coils 26 that are two-dimensionally arranged in a lattice form of five rows in the X direction and four columns in the Z direction. The number and arrangement of the RF receiving coils 26 are not limited to the example shown in Fig. 4.

As described above, the trunk part coil 22 has flexibility, and the plurality of RF receiving coils 26 are two-dimensionally arranged.

Returning to the description of Fig. 3, four loops 34, 36, 38, and 40 are disposed on a surface of the trunk part coil 22 on the +Y direction side (hereinafter, referred to as an "upper surface"). The loops 34, 36, 38, and 40 each comprise insertion holes 34A, 36A, 38A, and 40A (an example of a "plurality of through-holes") into which the belt 30 or the belt 32 is inserted. That is, the belts 30 and 32 are respectively passed through any of the insertion holes 34A, 36A, 38A, and 40A. The loops 34, 36, 38, and 40 function as restricting members that restrict the paths of the belts 30 and 32 inserted into the insertion holes 34A, 36A, 38A, and 40A, respectively. The insertion holes 34A, 36A, 38A, and 40A may be holes that penetrate the blanket 24 in the Y direction.

It is preferable that the loops 34, 36, 38, and 40 are disposed at the end of the rectangular long side of the trunk part coil 22 or the end of the rectangular short side of the trunk part coil 22. The end is not limited to the position of the rectangular-shaped side, and may be a position relatively close to the end to the extent that the end part of the trunk part coil 22 is in close contact with the subject P in a case where the trunk part coil 22 is fixed to the subject P by the belts 30 and 32.

In the example shown in Fig. 3, the loops 34, 36, 38, and 40 are disposed at the positions 22A, 22B, 22C, and 22D of the four corners of the trunk part coil 22, respectively. The four corners are not limited to the positions of the corners of the trunk part coil 22 and may be positions relatively close to the corners of the trunk part coil 22.

A stopper 42 is disposed on an opposite side of the subject P, with the trunk part coil 22 interposed therebetween. In the example shown in Fig. 3, the stopper 42 is disposed at a position 22E of a center of an upper surface of the trunk part coil 22 in a plan view. The center is not limited to the exact center, and may be a position relatively close to the center. The stopper 42 may be fixed to the position 22E of the trunk part coil 22, or may be provided separately from the trunk part coil 22.

The stopper 42 (an example of a "length adjustment mechanism") has a mechanism that fixes the belts 30 and 32 at positions of arbitrary lengths from the end parts 30A and 30B and the end parts 32A and 32B, respectively. The position of arbitrary length is not limited to an arbitrary position of continuous length, and may be a position of any of a plurality of positions of discrete lengths.

The stopper 42 comprises insertion holes 42A and 42B communicating from the +X direction side to the +Y direction side, insertion holes 42C and 42D communicating from the - X direction side to the +Y direction side, and a locking mechanism (not shown) capable of adjusting opening amounts of the insertion holes 42A, 42B, 42C, and 42D. The insertion holes 42A, 42B, 42C, and 42D may be arranged on a straight line parallel to the X direction on the +Y direction side.

In the locking mechanism, the stopper 42 relatively increases the opening amount of the insertion holes 42A, 42B, 42C, and 42D in the unlocked state, and the belts 30 and 32 are inserted into the insertion holes 42A, 42B, 42C, and 42D so as to be movable. In addition, in a case where the locking mechanism is in the locked state, the stopper 42 relatively reduces the opening amount of the insertion holes 42A, 42B, 42C, and 42D and restricts the movement of the belts 30 and 32 inserted into the insertion holes 42A, 42B, 42C, and 42D. That is, the stopper 42 can fix the belts 30 and 32 at positions of arbitrary lengths.

In the example shown in Fig. 3, in the belt 30, the end part 30A is inserted into the insertion hole 42A of the stopper 42 through the insertion hole 34A of the loop 34, and the end part 30B is inserted into the insertion hole 42D of the stopper 42 through the insertion hole 40A of the loop 40. The belt 30 passes between the loop 34 and the loop 40 over the -Y direction side of the trunk part coil 22. In addition, in the belt 32, an end part 32A is inserted into the insertion hole 42B of the stopper 42 through the insertion hole 36A of the loop 36, and an end part 32B is inserted into the insertion hole 42C of the stopper 42 through the insertion hole 38A of the loop 38. The belt 32 passes between the loop 36 and the loop 38 over the -Y direction side of the trunk part coil 22 and intersects the belt 30.

In order to put the trunk part coil 22 on the examination target part of the subject P (see Fig. 1), the technician first disposes the examination target part of the subject P between the belt 30 and 32 and the surface of the trunk part coil 22 of the coil set 10 on the -Y direction side in the state shown in Fig. 3. For example, in a case where the examination target part is the abdomen, the coil set 10 in the state shown in Fig. 3 is put on the subject P from the head, the trunk part coil 22 is disposed on the abdomen side, and the belts 30 and 32 are disposed on the back side.

Next, the technician disposes the subject P on the top plate 118 (see Fig. 1). For example, in a case where the examination target part is the abdomen, the subject P is caused to lie on his/her back on the top plate 118 in a state where the trunk part coil 22 is disposed on the abdomen side.

Then, the technician sets the locking mechanism of the stopper 42 to the unlocked state, and pulls the end parts 30A and 30B of the belt 30 and the end parts 32A and 32B of the belt 32, which are protruding from the +Y direction side of the stopper 42 of the trunk part coil 22, in the +Y direction to bring the trunk part coil 22 into close contact with the abdomen of the subject P.

Finally, the technician sets the locking mechanism of the stopper 42 to the locked state in a state where the trunk part coil 22 is in close contact with the abdomen of the subject P, and fixes the belts 30 and 32. Therefore, the belts 30 and 32 are adjusted to arbitrary lengths. In this way, the technician can complete the fixation of the trunk part coil 22 by pulling the belts 30 and 32 at positions on the surface of the trunk part coil 22 that are easy to grab and easy to hold.

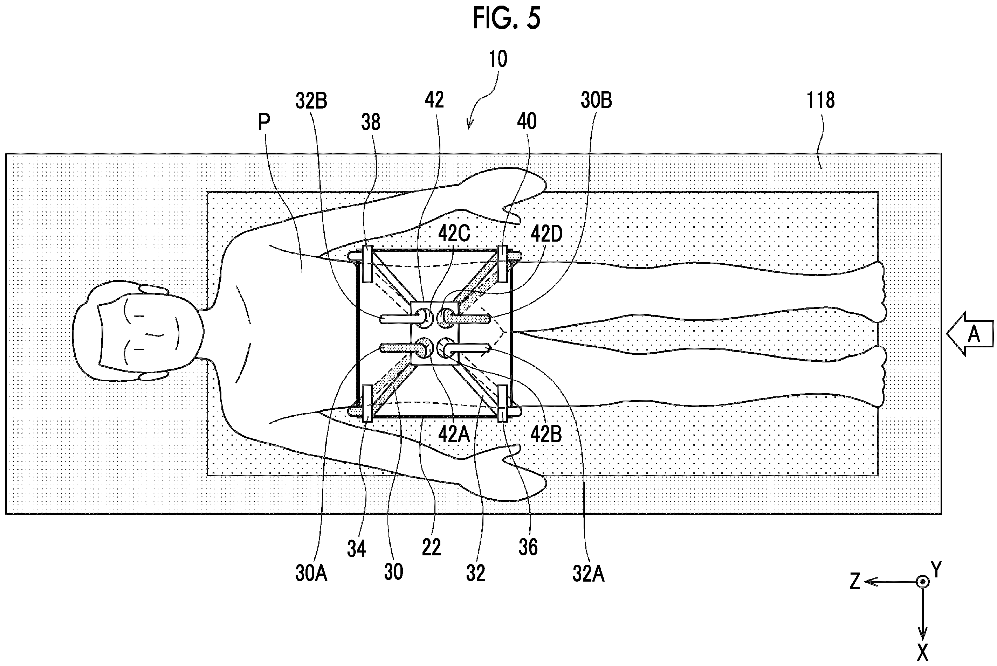

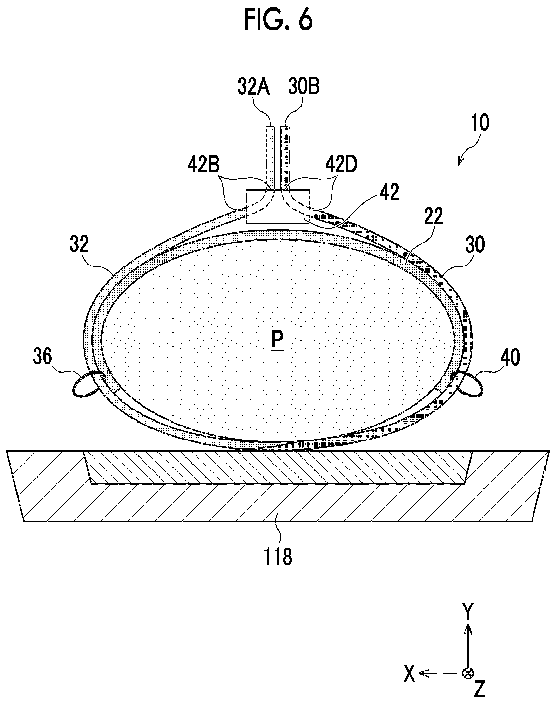

Fig. 5 is a top view of the trunk part coil 22 put on the abdomen of the subject P. In Fig. 5, the subject P is placed on the top plate 118, and the belts 30 and 32 on the back side of the subject P are indicated by dotted lines. Fig. 6 is a schematic view viewed from the arrow A of Fig. 5.

As shown in Fig. 5, the belts 30 and 32 intersect behind the subject P, and thus the entire trunk part coil 22 can be brought into close contact with the subject P without a gap as shown in Fig. 6. Therefore, the RF receiving coil 26 can receive the nuclear magnetic resonance signal with relatively high sensitivity and can perform imaging with relatively high quality. The breathing synchronized imaging synchronized with the breathing of the subject P can be performed by breathing sensing using a camera (not shown) disposed in the examination space 114 (see Fig. 1).

In addition, since there is no fixing point between the belts 30 and 32 and the trunk part coil 22, the belts 30 and 32 on the back side of the subject P can fix the trunk part coil 22 without moving. Therefore, it is not necessary to provide a guide for sliding the belts 30 and 32 on the back side of the subject P, and the comfort of the subject P in case of lying is improved, and it is possible to prevent the discomfort of the subject P such as the movement of the belts 30 and 32 on the back side of the subject P.

In the examples shown in Figs. 5 and 6, the trunk part coil 22 is put on the subject P such that the short side of the rectangular shape is aligned in the direction along the body axis of the subject P, but the trunk part coil 22 may be put on the subject P such that the long side of the rectangular shape is aligned in the direction along the body axis of the subject P. As described above, the trunk part coil 22 can be fixed to the subject P in both a vertically long direction (an example of an orientation in which a long side of a rectangular shape is aligned with an axis of an examination target part of the subject) and a horizontally long direction (an example of an orientation in which a short side of a rectangular shape is aligned with an axis of an examination target part of the subject). The technician may input whether the trunk part coil 22 is fixed in a vertically long direction or a horizontally long direction to a signal processing device (not shown).

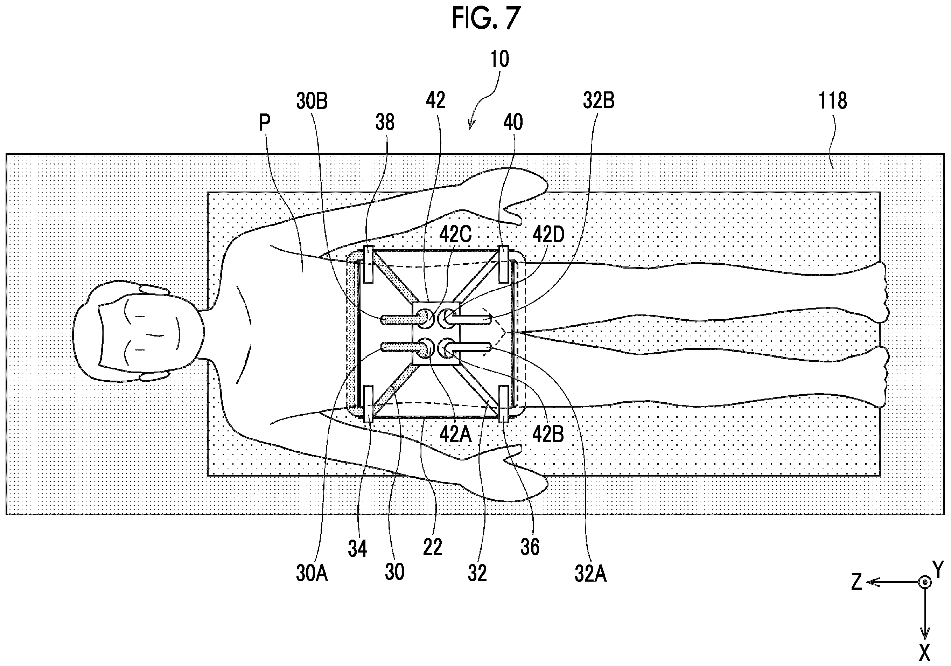

Fig. 7 is a top view of the trunk part coil 22 put on the abdomen of the subject P by another method. In the example shown in Fig. 7, in the belt 30, the end part 30A is inserted into the insertion hole 42A of the stopper 42 through the insertion hole 34A of the loop 34, and the end part 30B is inserted into the insertion hole 42C of the stopper 42 through the insertion hole 38A of the loop 38. The belt 30 is passed between the loop 34 and the loop 38 over the back surface of the subject P.

In addition, in the belt 32, the end part 32A is inserted into the insertion hole 42B of the stopper 42 through the insertion hole 36A of the loop 36, and the end part 32B is inserted into the insertion hole 42D of the stopper 42 through the insertion hole 40A of the loop 40. The belt 32 is passed between the loop 36 and the loop 40 over the back surface of the subject P.

As described above, the belts 30 and 32 may fix the trunk part coil 22 to the subject P without intersecting each other. In the example shown in Fig. 7, the trunk part coil 22 is fixed to the subject P in a horizontally long state. However, in a case where the trunk part coil 22 is fixed to the subject P in a vertically long state, it is necessary to reinsert the belts 30 and 32 into the insertion holes 36A, 38A, 40A, and 42A of the loops 36, 38, 40, and 42. On the other hand, in the example shown in Fig. 5, in both a case where the trunk part coil 22 is fixed to the subject P in a vertically long state and a case where the trunk part coil 22 is fixed to the subject P in a horizontally long state, the insertion holes 36A, 38A, 40A, and 42A through which the belts 30 and 32 are passed may be fixed. Therefore, it is preferable that the belts 30 and 32 intersect behind the subject P.

### [Second Embodiment]

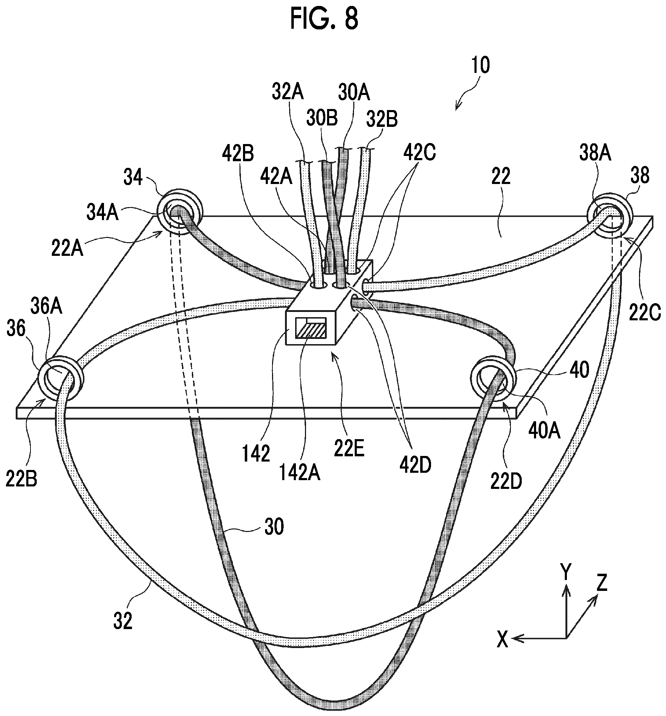

Fig. 8 is a perspective view of the coil set 12 according to a second embodiment. As shown in Fig. 8, a stopper 142 is disposed at the position 22A of the center of the upper surface of the trunk part coil 22 of the coil set 12.

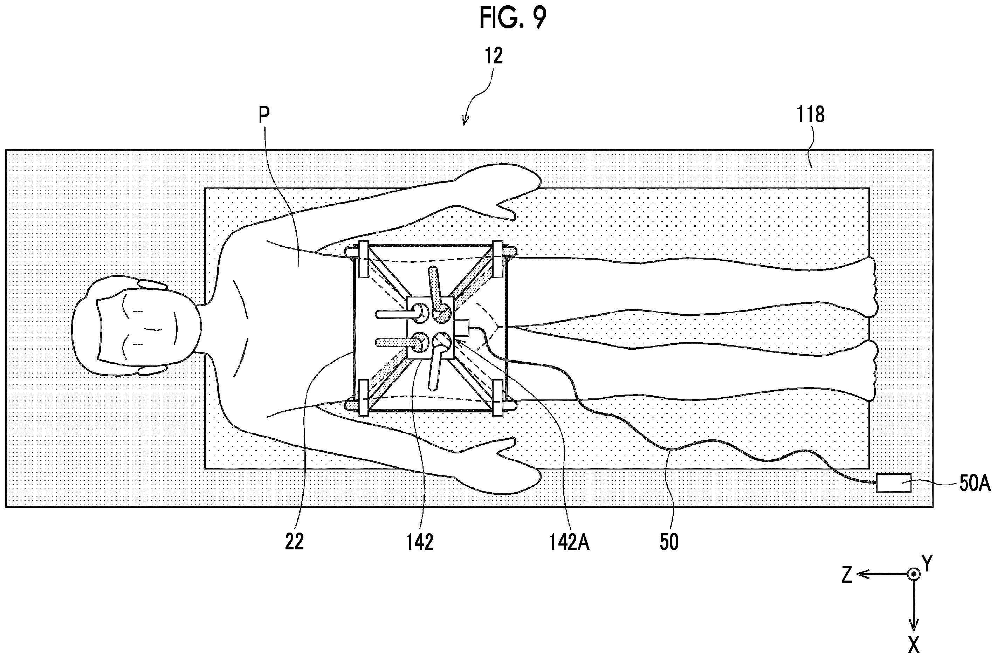

The stopper 142 comprises the insertion holes 42A, 42B, 42C, and 42D and a relay connector 142A. The relay connector 142A is a connector for connecting a reception cable 50 (see Fig. 9) that outputs a nuclear magnetic resonance signal received by the plurality of RF receiving coils 26 (see Fig. 4).

A signal detection circuit (not shown) is connected to each of the plurality of RF receiving coils 26. The output of each of the plurality of signal detection circuits is connected to each terminal of the relay connector 142A. The nuclear magnetic resonance signal received by the RF receiving coil 26 is detected by the signal detection circuit and is transmitted to the relay connector 142A.

Fig. 9 is a top view of the trunk part coil 22 put on the abdomen of the subject P. As shown in Fig. 9, the reception cable 50 is connected to the relay connector 142A of the stopper 142. A receiving-side connector 50A is connected to the other end of the reception cable 50. An examination table cable (not shown) is connected to the receiving-side connector 50A. As a result, the nuclear magnetic resonance signal received by the RF receiving coil 26 is output to a signal processing device (not shown) via the relay connector 142A, the reception cable 50, and the examination table cable.

According to the coil set 12, the technician can efficiently perform two operations, that is, an operation of fixing the trunk part coil 22 to the subject P and an operation of connecting the trunk part coil 22 and the reception cable 50, at the same position.

A wireless communication device using a predetermined protocol may be provided to the stopper 142 instead of the relay connector 142A, and the nuclear magnetic resonance signal received by the RF receiving coil 26 may be output in the wireless communication device by wireless communication.

### [Third Embodiment]

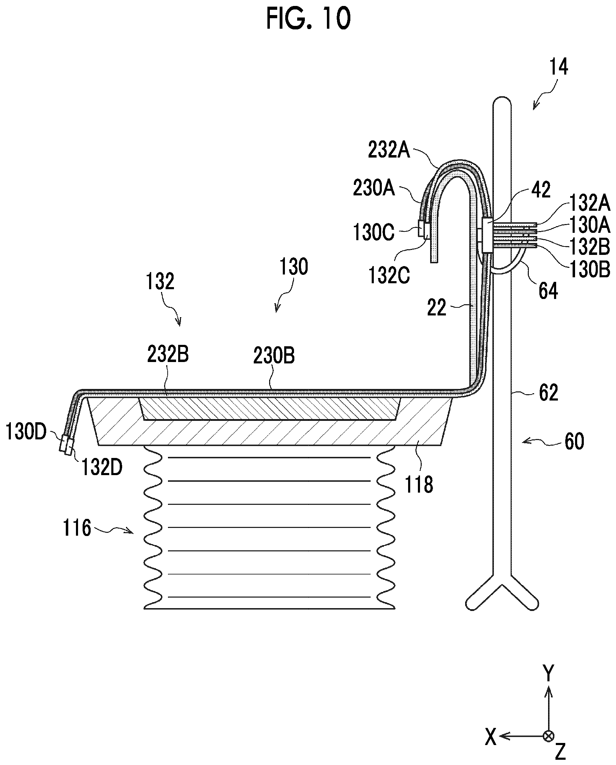

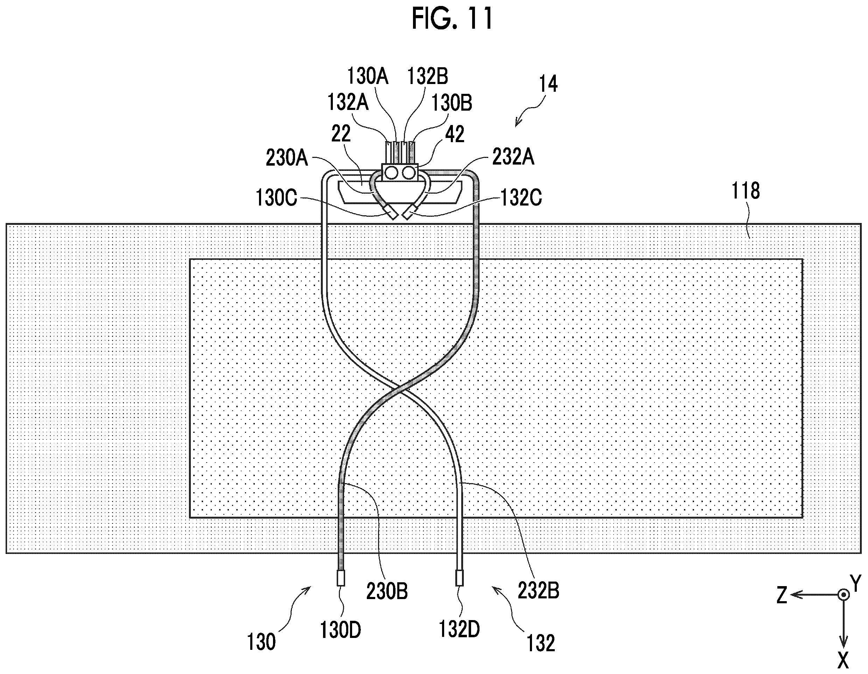

Fig. 10 is a front view of a coil set 14 according to a third embodiment. In addition, Fig. 11 is a top view of the coil set 14. As shown in Fig. 10, the coil set 14 comprises a support base 60 at the position on the -X direction side of the examination table 116. The support base 60 comprises a shaft portion 62 and a hook portion 64. In Fig. 11, the support base 60 is not shown.

The shaft portion 62 is a support column extending in the Y direction. The hook portion 64 is fixed to the shaft portion 62. The hook portion 64 is a U-shaped locking member for hooking and holding the trunk part coil 22. In the examples shown in Figs. 10 and 11, the trunk part coil 22 is held by the support base 60 with the stopper 42 hooked to the hook portion 64. It is preferable that the hook portion 64 is disposed on the +Y direction side with respect to the top plate 118.

In addition, the coil set 14 comprises two belts 130 and 132. The two belts 130 and 132 are configured separately from the trunk part coil 22. The belt 130 comprises an end part 130A and an end part 130B, which are a free ends, respectively. The belt 130 comprises a belt member 230A (an example of a "first belt member") and a belt member 230B (an example of a "second belt member"). The belt member 230A comprises a connector 130C of which one end is an end part 130A and one of a pair of connectors (an example of a "pair of connecting members") is at the other end. The belt member 230B comprises a connector 130D of which one end is an end part 130B and the other of the pair of connectors is at the other end. The belt member 230A and the belt member 230B are connected by the connector 130C and the connector 130D to form one belt 130.

The belt 132 comprises end parts 132A and 132B that are free ends, respectively. The belt 132 comprises a belt member 232A (an example of a "first belt member") and a belt member 232B (an example of a "second belt member"). The belt member 232A comprises a connector 132C of which one end is the end part 132A and one of a pair of connectors is at the other end. The belt member 232B comprises a connector 132D of which one end is the end part 132B and the other of the pair of connectors is at the other end. The belt member 232A and the belt member 232B are connected by the connector 132C and the connector 132D to form one belt 132.

The connectors 130C and 130D, and the connector 132C and the connector 132D may be a member that is easily attachable and detachable, such as a pair of hooks which are engaged to each other, a surface fastener, or a buckle.

The end part 130A and the end part 130B of the belt 130 and the end part 132A and the end part 132B of the belt 132 are inserted into the insertion holes 42A, 42B, 42C, and 42D (not shown in Figs. 10 and 11, see Fig. 3) of the stopper 42, respectively.

As shown in Figs. 10 and 11, in the belt 130 from which the connection of the connector 130C and the connector 130D is disconnected, the belt member 230B is inserted into the insertion hole 40A of the loop 40 (not shown in Figs. 10 and 11, see Fig. 3) and is placed on the top plate 118 (+ Y direction side) from the end on the -X direction side to the end on the +X direction side. Similarly, in the belt 132 from which the connection of the connector 132C and the connector 132D is disconnected, the belt member 232B is inserted into the insertion hole 38A of the loop 38 (not shown in Figs. 10 and 11, see Fig. 3) and is placed on the top plate 118 from the end on the -X direction side to the end on the +X direction side.

In addition, as shown in Fig. 11, the belt member 230B and the belt member 232B are placed on the top plate 118 so as to intersect each other. That is, on the end of the top plate 118 on the -X direction side, among the belt member 230B and the belt member 232B, the belt member 230B is disposed on the -Z direction side and the belt member 232B is disposed on the +Z direction side. On the other hand, on the end of the top plate 118 on the +X direction side, among the belt member 230B and the belt member 232B, the belt member 230B is disposed on the +Z direction side and the belt member 232B is disposed on the -Z direction side.

As shown in Figs. 10 and 11, in order to put the trunk part coil 22 on the subject P in a state where the coil set 14 is disposed, the technician first makes the subject P lie on his/her back on the top plate 118. Next, the technician places the trunk part coil 22 held by the support base 60 on the abdomen, which is the examination target part of the subject P. Subsequently, the technician inserts the belt member 230B into the insertion hole 34A of the loop 34 (not shown in Figs. 10 and 11, see Fig. 3) from the connector 130D side and connects the connector 130C and the connector 130D. As a result, the belt member 230A and the belt member 230B are integrated with each other to form one belt 130.

Similarly, the technician inserts the belt member 232B into the insertion hole 36A of the loop 36 (not shown in Figs. 10 and 11, see Fig. 3) from the connector 132D side and connects the connector 132C and the connector 132D. As a result, the belt member 232A and the belt member 232B are integrated to form one belt 132.

Further, the technician sets the locking mechanism of the stopper 42 to the unlocked state, and pulls the end parts 130A and 130B of the belt 130 and the end parts 132A and 132B of the belt 132, which are protruding from the +Y direction side of the stopper 42 of the trunk part coil 22, in the +Y direction to bring the trunk part coil 22 into close contact with the abdomen of the subject P.

Finally, the technician sets the locking mechanism of the stopper 42 to the locked state in a state where the trunk part coil 22 is in close contact with the abdomen of the subject P, and fixes the belts 130 and 132. As described above, the trunk part coil 22 is put on the subject P.

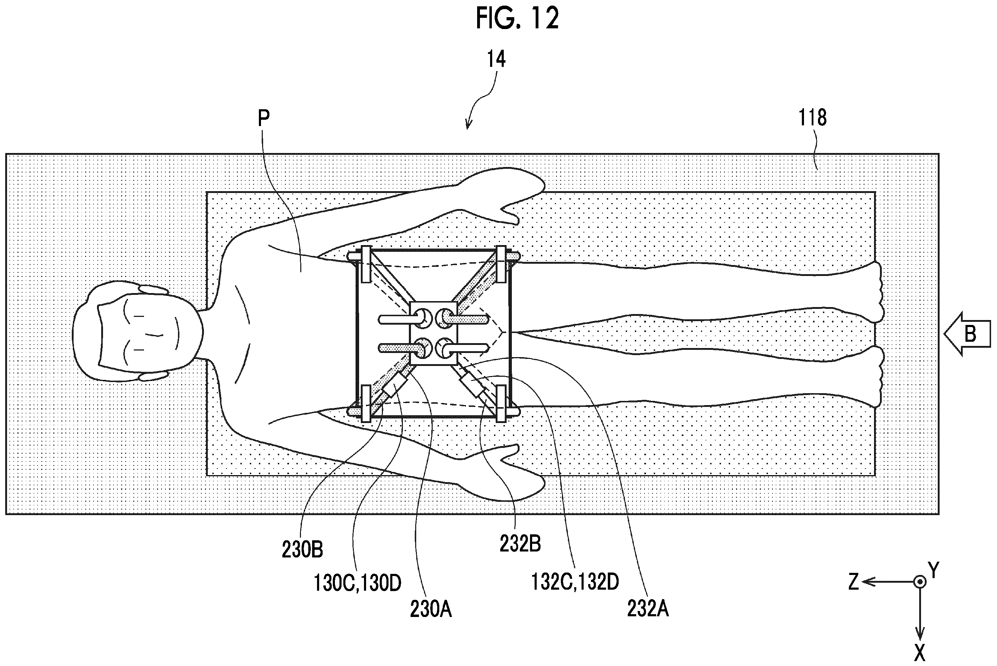

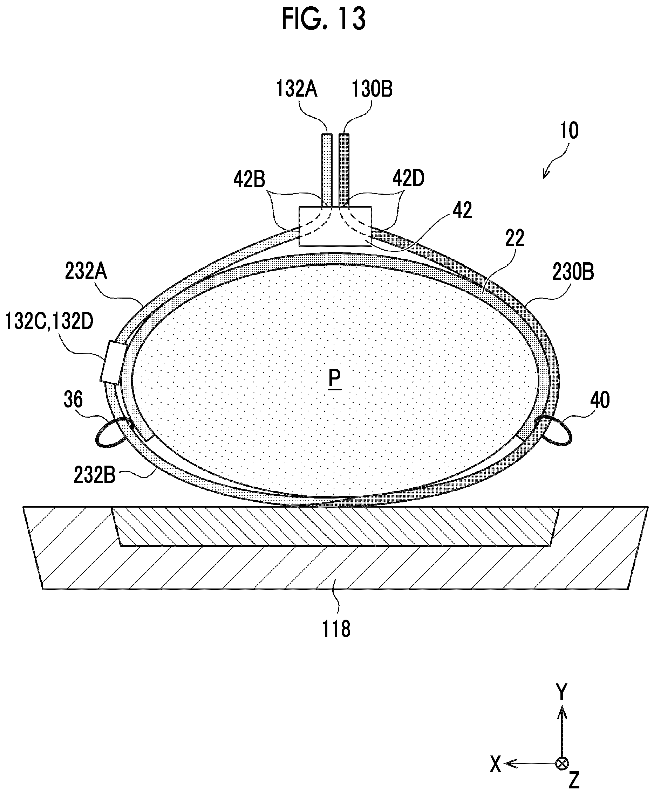

Fig. 12 is a top view of the trunk part coil 22 put on the abdomen of the subject P. In addition, Fig. 13 is a schematic view viewed from the arrow B of Fig. 12. As shown in Figs. 12 and 13, the technician can closely attach and put the trunk part coil 22 to the subject P.

With the coil set 14, it is not necessary for the subject P to put on the trunk part coil 22 before the subject P is placed on the top plate 118, and the workflow can be improved. In addition, even in a case where the trunk part coil 22 is removed from the subject P, a motion of taking off the trunk part coil 22 from the head side of the subject P is not necessary. In a case where the trunk part coil 22 is removed, the subject P can be released by disconnecting the connector 130C and the connector 130D and disconnecting the connector 132C and the connector 132D. As a result, a breathing synchronization sensor (not shown) that detects the breathing of the subject P and is installed between the subject P and the trunk part coil 22 can be installed on the examination target part of the subject P lying on the top plate 118 in the same manner as in the related art, and then the trunk part coil 22 can be installed.

### [Others]

The technical scope of the present invention is not limited to the scope according to the embodiment described above. The configurations and the like in each embodiment can be appropriately combined between each of the embodiments without departing from the scope of the present invention.

### Explanation of References

100: MRI apparatus
112: gantry
114: examination space
116: examination table
118: top plate
122: fixing belt
P: subject

## Claims

1. A coil set (10, 12, 14) comprising:
a trunk part coil (22) that has flexibility and in which a plurality of receiving coils (26) that receive a nuclear magnetic resonance signal of a subject are two-dimensionally arranged;
two belts (30, 32, 130, 132) that are configured separately from the trunk part coil (22) and that fix the trunk part coil (22) to the subject; and
a length adjustment mechanism (42, 142) that fixes the two belts (30, 32, 130, 132) at positions of arbitrary lengths,
wherein the trunk part coil (22) includes a plurality of through-holes (34A, 36A, 38A, 40A), and
the two belts (30, 32, 130, 132) are each passed through any of the plurality of through-holes (34A, 36A, 38A, 40A), and the length adjustment mechanism (42, 142) adjusts lengths of the two belts (30, 32, 130, 132) to arbitrary lengths.

2. The coil set (10, 12, 14) according to claim 1,
wherein the two belts (30, 32, 130, 132) intersect each other to fix the trunk part coil (22) to the subject.

3. The coil set (10, 12, 14) according to claim 1 or 2,
wherein the trunk part coil (22) includes four through-holes (34A, 36A, 38A, 40A).

4. The coil set (10, 12, 14) according to claim 3,
wherein the trunk part coil (22) includes the through-holes (34A, 36A, 38A, 40A) at four corners of the trunk part coil (22) in a plan view.

5. The coil set (10, 12, 14) according to any one of claims 1 to 4,
wherein the length adjustment mechanism (42, 142) is disposed on an opposite side of the subject, with the trunk part coil (22) interposed therebetween.

6. The coil set (10, 12, 14) according to claim 5,
wherein the length adjustment mechanism (42, 142) is fixed to a center of the trunk part coil (22) in a plan view.

7. The coil set (12) according to any one of claims 1 to 6,
wherein the length adjustment mechanism (142) includes a relay connector (142A) to which a cable (50) that outputs a nuclear magnetic resonance signal received by the plurality of receiving coils (26) is connected.

8. The coil set (14) according to any one of claims 1 to 7,
wherein each of the two belts (130, 132) includes
a first belt member (230A) that includes one of a pair of connecting members (130A), and
a second belt member (230B) that includes the other of the pair of connecting members (130B).

9. The coil set (14) according to claim 8, further comprising:
a breathing synchronization sensor that is disposed between the trunk part coil (22) and the subject and that detects breathing of the subject.

10. The coil set (10, 12, 14) according to any one of claims 1 to 9,
wherein the trunk part coil (22) has a rectangular shape in a plan view, and is configured to fix the subject in any of an orientation in which a long side of the rectangular shape is aligned with an axis of an examination target part of the subject and an orientation in which a short side of the rectangular shape is aligned with the axis of the examination target part of the subject.
